Europäisches Patentamt

European Patent Office

Office européen des brevets

(11) Publication number: **0 455 255 A1**

# EUROPEAN PATENT APPLICATION

(21) Application number: 91107200.7

(22) Date of filing: 03.05.91

(51) Int. Cl.⁵: **A61B 17/58**

(30) Priority: 04.05.90 PL 285079

(43) Date of publication of application:
06.11.91 Bulletin 91/45

(84) Designated Contracting States:
AT CH DE FR LI

(71) Applicant: **Ramotowski, Witold**
ul. Irysowa 10b
PL-02-660 Warszawa(PL)

Applicant: **Granowski, Robert**
ul. Wiertnicza 53
PL-00-952 Warszawa(PL)

Applicant: **Cieplak, Jerzy**
ul. Mickiewicza 28/2
PL-41-300 Dabrowa Gornicza(PL)

Applicant: **Paszkowski, Marek**

ul. Toszecka 144
PL-44-100 Gliwice(PL)

(72) Inventor: **Ramotowski, Witold**
ul. Irysowa 10b
PL-02-660 Warszawa(PL)
Inventor: **Granowski, Robert**
ul. Wiertnicza 53
PL-00-952 Warszawa(PL)
Inventor: **Cieplak, Jerzy**
ul. Mickiewicza 28/2
PL-41-300 Dabrowa Gornicza(PL)
Inventor: **Paszkowski, Marek**
ul. Toszecka 144
PL-44-100 Gliwice(PL)

(74) Representative: **Füchsle, Klaus, Dipl.-Ing. et al**
**Hoffmann . Eitle & Partner Patentanwälte**
**Arabellastrasse 4**
**W-8000 München 81(DE)**

(54) A plate-type bone stabilizer.

(57) The invention relates to a bone stabilizer comprising bone screws (3) and a support plate (1) which is provided with a narrowing in the form of a rounded recess, the support plate (1) connecting the free fragments of the bone by means of clamping disks (2). The clamping disks (2) are rigid connectors for the bone screws (3), the heads (14) of which are in the form of the two truncated cones connected at their bases. The heads (14) of the bone screws (3) are mounted into appropriately formed apertures and respectively of the support plate (1) and the clamping disks (2).

Fig.2

EP 0 455 255 A1

The subject of the present invention is a plate-type bone stabilizer. The said stabilizer is applicable in the plasterless surgical treatment of bones.

There are known a few kinds of sets which serve to surgically restore and ensure the adequate stabilization of chipped bones. The best known is the Osteosynthesis Association set which is suited for the plate-type osteosynthesis. The O.A. set comprises a rectangle-shaped plate with round sockets in their upper part being in the form of truncated cone, bone screws consisting of a circular-base or truncated-cone head having the angle of flare of 45°, and a bone thread. Another variation of the O.A. set comprises a rectangle-shaped self-compression plate, wherein from one side there are round sockets being in the form of a truncated cone, whereas from the other side there are oval sockets serving as compression holes which have their upper part truncated along the entire perimeter thereof or they are one-sidedly truncated, and a bone theread. The anastomosis by means of the O.A. set consists in fastening the said plate directly to chipped bones by mean of bone screws. The stabilization of the chipped bone is attained by compressing the screws into the compression holes of the plate, whereas the usable force axially compressing the chipped bones is a function of the compression forces upon the bones being not usable and harmful for the anastomosis itself. The damages to the threading of the bone and the osteolysis along the adhesion area of the plate result in the drop of forces which stabilize the chipped bone, what sometimes causes the mobility of the chipped bone and finally the disturbances in growing together of the bone. Another hitherto known set is a self-compression bone anastomosis set which is inter alia known from the Polish patent specification No. 145.631. This set comprises a self-compression rectangle-shaped plate, wherein there are oval sockets having their upper part in the form of a truncated cone, and bone screws being in their intraosseous part provided with a cortex thread which is separated from the part with a metric thread by means of a square-shapped thickening of the size being bigger than a slit into the aperture of the oval socket of the plate.

The screws are screwed into the bone in such a manner that the thickenings do not touch it, whereafter a self-compression plate is put there-upon and nuts are tightened up upon the metric thread, what results in its bending aside within the elasticity limit.

The above-described set, although undeniably advantageous over the formerly discussed O.A. set has some defficiencies in its design, which result in the too low flexibility of the anastomosed joint, especially above the slit of the fractured bone; in the loosening up of nuts upon the metric thread of bone screws what destabilizes the anastomozed joint; in the cracking up of bone screws above a thrust disk due to the "notch" effect, in the occurrence of varied stresses in the chipped bone due to separately screwing in of nuts upon the metric thread of bone screws, and in the lack of any possiblity to correct the compression of the chipped bone without being it necessary to disassemble the plate.

The aim of the present invention was to work out the design of a stabilizer which would maintain the advantages of the hitherto stabilizers with eliminating their defficiences, and most of all the design which would ensure the appropriate stress above the slit of the fractured bone , the stability of the system as well as the possibility of correcting the compression of the chipped bone. The essence of the present invention consists in the fact that a support plate of the bone stabilizer is at its half-length provided with a rounded recess which diminishes the width above the slit of the fractured bone. The support plate joins free fragments of the bone with the aid of clamping disks by resting there-upon and on the heads of bone screws. The clamping disks, being connected to the support disks by means of joining screws, are mounted into the adequately shaped recesses at the concave side of the support disk and they constitute a rigid connector with the bone screws. The length of recesses into the support disk is bigger than the length of the clamping disk by the length of a segment whereby they the disks are mutually displaceable.

The heads of the bone screws are in the form of two truncated cones of the varied angle of flare, being turned to each other with their bases. It is preferable that the head of the screw be provided with a small roll between said truncated cones. The heads of the bone screws are mounted into the adequately formed apertures of the support plate and of the clamping disks. The said apertures are in the form of the two different truncated cones.

In case of complicated fractures with inflammatory focuses it is possible to have a system of the two or more plate-type bone stabilizers as to move away the bone screws from the slit of the fractured bone, being connector's clamping disk which is at its half-length, similarly as the support plate, provided with a rounded recess which diminishes the length thereof. Such a design of the plate-type bone stabilizer enables to maintain the elasticity above the slit of the fractured bone, whereas it enables to attain high rigidity in the area above the bone fragments. The design of the stabilizer makes it possible that the clamping disk is displaceable relative to the support plate due to the appropriate formation of the heads of bone screws and of the apertures into the support plate and clamping disks.

It is possible to displace the said disk and the same time to control the compression of the bone with the aid of connecting screws, without being it necessary to disassemble the plate-type bone stabilizer. The said stabilizer is finally positioned relative to the chipped bone by means of the bone screws owing to the fact that it is possible to find access to their sockets through the apertures in the upper part of the support plate. The subject of the present invention has been shown into the example of embodiment, wherein in Fig. 1 there is shown the plate-type bone stabilizer in an axonometric projection, in Fig. 2 the assembled plate-type bone stabilizer is shown in a cross-section together with the distribution of forces into the anastomosed joint, in Fig. 3 the support plate is shown in a top view, in Fig. 4 the support plate is shown in its narrowed portion in a cross-section, in Fig. 5 the support plate is shown in its recessed portion in a cross-section, in Fig. 6 the clamping disk is shown in a top view, in Fig. 7 a bone screw is shown in a view with a broken-out section, in Fig. 8 a connector's clamping disk is shown in a top view, in Fig. 9 a system of the two bone stabilizers as mounted into the chipped bone is shown, and in Fig. 10 a system of the two bone stabilizers in a top view is shown.

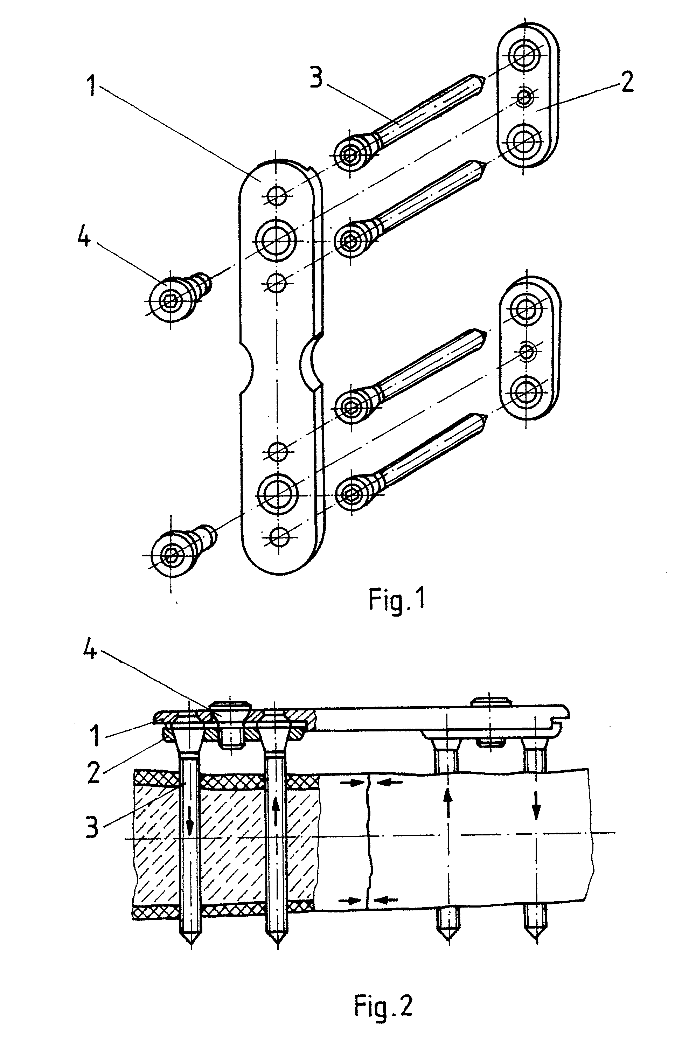

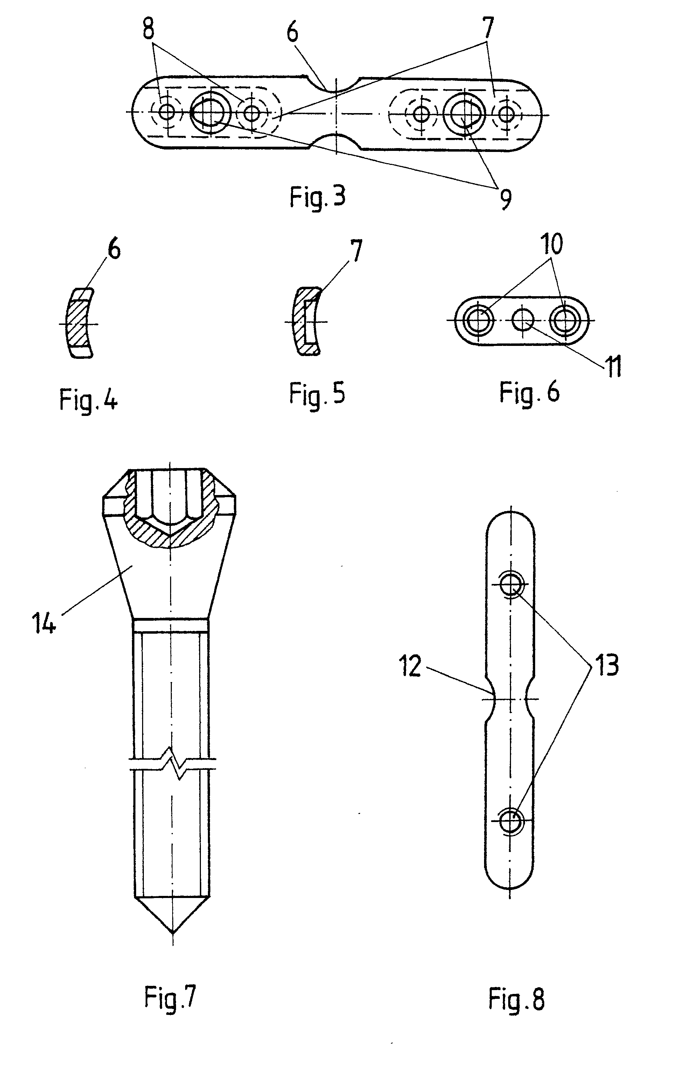

The plate-type bone stabilizer comprises a support plate 1, clamping disks 2, bone screws 3 and connecting screws 4. Support plate 1 as shown in Figs 1, 2, 3, 4, 5, connecting the free fragments of the bone with the aid of the two clamping disks 2 being shown in Fig. 6 is provided with a characteristic narrowing being in the form of a rounded recess 6. Support plate 1 has in its lower sliglitly concave portion the two recesses 7 covering with its length the apertures for bone screws 3 and for connecting screws 4, and terminating with a rounded end just before the more flexible portion of the plate. The four apertures 8 for bone screws 3 in support plate 1 as shown in Fig. 7 are adjusted to the shape of the heads of the bone screws in such a manner that they are in the form of truncated cones of the angle of flare of 90° being with their bases directed towards recesses 7, wherein there are placed clamping disks 2. Between said apertures 8 there are placed oval apertures 9 of oblique edges for connecting screws 4. Clamping disks 2, being a rigid member which connects the bone screws 3 adjoining to a given bone fragment, are connected by means of connecting screws 4 to support plate 1, and with their flat portion they touch the surface of recesses 7 into support plates 1. The two apertures 10 for bone screws 3 are in the form of truncated cones of the angle of flare of 30°,being with their bases directed towards support plate 1. Between apertures 10 there are placed threaded apertures 11 suited for connecting screws 4.

Bone screw 3 as illustrated in Fig. 7 is provided with a head 14 in the form of the two truncated cones of the angle of flare of 90° for the upper one and 30° for the lower one, being with their bases connected together via a segment of the cylinder of the base being equal to the bases of the cones. The profile of the threading of the bone screws is dependable on the nature of the bone fragments being connected together.

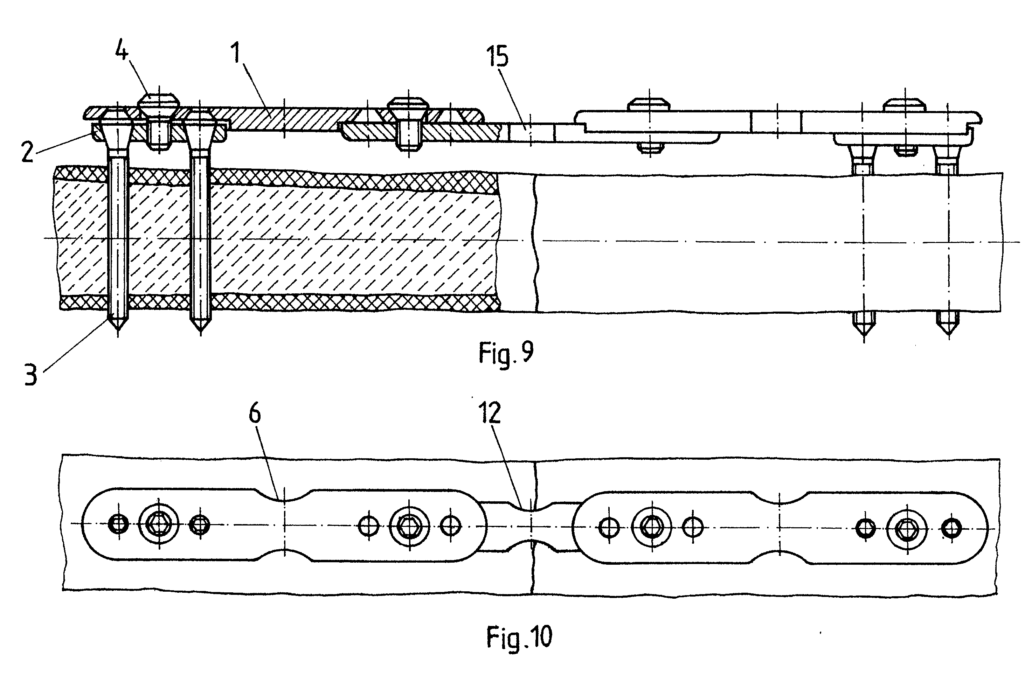

The connector's clamping disk 15 as shown in Fig. 8, 9 and 10 is in its half-length provided with a narrowing 12 and with the two threaded apertures 13 which serve to connect said disk to support plate 1 by means of connecting screws 4 which have low-pitch metric threading. The bone screws as well as the connecting screws are provided with identical hexagonal sockets of the size of 3.5 mm, and the depth of 3.5 mm.

All the members of the said stabilizer are made of the biocompatible quality of steel. All the surfaces of the said members are mechanically and electrolytically polished with a passive layer applied.

The bone stabilizer as per the present invention enables the inner and outer osteosynthesis be attained with making it possible that compression joints are achieved , and that it is effected without the axial compression of the chipped bone.

## Claims

1. A plate-type bone stabilizer for the surgical treatment of fractured bones by the stabilization of bone fragments, comprising a support plate for connecting the fragments and bone screws, characterized in that the support plate is provided with a narrowing which reduces the width thereof, that the support plate connects the free fragments of the bone via clamping disks which respectively constitute rigid connectors to the bone screws, the claimping disks respectively being connected to the support plate by means of connecting screws, and that the heads of the bone screws are in the form of two truncated cones connected by their respective bases, the heads of the bone screws being mountable in appropriately truncated cone-shaped apertures respectively provided in the support plate and the clamping disks.

2. Plate-type bone stabilizer according to claim 1, characterized in that the clamping disks connected to the support plate are mounted in recesses provided in the support plate.

3.  Plate-type bone stabilizer according to claim 1, characterized in that the two truncated cones forming the head of the bone screws have different flare angles.

4.  Plate-type bone stabilizer according to claim 1, characterized in that the bases of the two truncated cones forming the head of the bone screws are connected by a short cylindrical segment.

5.  Plate-type bone stabilizer according to claim 1, characterized in that the narrowing is in the form of a rounding recess.

6.  Plate-type bone stabilizer according to claim 1, characterized in that the apertures in the support plate and the clamping disks have respectively different truncated cone shapes.

7.  Plate-type bone stabilizer according to claim 1, characterized in that it constitutes a system of two or more stabilizers which are connected by means of connecting screws and a connecting clamping disk, the connecting clamping disk being provided at its half-length with a narrowing in the form of a rounded recess.

Fig.1

Fig.2

Fig. 3

Fig. 4

Fig. 5

Fig. 6

Fig. 7

Fig. 8

Fig. 9

Fig. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 683 878 (CARTER)<br>* column 3, line 58 - column 4, line 19; figure 2 *<br>— — — | 1 | A 61 B 17/58 |
| Y | EP-A-0 201 024 (WOLTER)<br>* page 9, line 23 - page 10, line 4; claim 1; figure 4 *<br>— — — | 1 | |
| A | FR-A-2 556 583 (I.N.S.E.R.M.)<br>* page 7, line 14 - page 7, line 21; figures 1,2 *<br>— — — — — | 1 | |

|  |
|---|
| TECHNICAL FIELDS SEARCHED (Int. Cl.5) |
| A 61 B |

The present search report has been drawn up for all claims

| Place of search | Date of completion of search | Examiner |
|---|---|---|
| The Hague | 05 August 91 | MOERS R.J. |